# EUROPEAN PATENT SPECIFICATION

(11) **EP 1 980 065 B1**
(45) Date of publication and mention of the grant of the patent: **24.05.2017**
(21) Application number: 07709981.0
(22) Date of filing: 08.01.2007
(51) Int. Cl.: A61B 5/00, G06F 19/00, H04W 12/06, H04W 84/18

(54) **AUTOMATIC AND SECURE CONFIGURATION OF WIRELESS MEDICAL NETWORKS**
AUTOMATISCHE UND SICHERE KONFIGURATION VON DRAHTLOSEN NETZWERKEN
CONFIGURATION AUTOMATIQUE ET SÉCURISÉE DE RÉSEAUX MÉDICAUX SANS FIL

(30) Priority: 18.01.2006 US 760020 P
(43) Date of publication of application: 15.10.2008
(73) Proprietor: Koninklijke Philips N.V., 5656 AE Eindhoven (NL)
(72) Inventor: BALDUS, Heribert, D-52076 Aachen (DE); KLABUNDE, Karin, D-44795 Bochum (DE); GALLO, Francesco, D-52076 Aachen (DE)
(74) Representative: de Haan, Poul Erik
(86) International application number: PCT/US2007/060204
(87) International publication number: WO 2007/084807

(56) References cited:
- EP-A- 1 024 626
- EP-A- 1 096 722
- EP-A- 1 220 501
- WO-A-2006/087670
- US-A1- 2003 125 017

## Description

The following relates to the network systems and methods. It finds particular application in conjunction with short-range medical wireless network systems and will be described with particular reference thereto. However, it is to be appreciated that the following will also find application in conjunction with other network systems and the like.

Short-range wireless systems typically have a range of less than one hundred meters, but may connect to the Internet to provide communication over longer distances. Short-range wireless systems include, but are not limited to, a wireless personal area network (PAN) and a wireless local area network (LAN). A wireless PAN uses low-cost, low-power wireless devices that have a typical range of about ten meters. An example of a wireless PAN technology is the IEEE 802.15.1 Bluetooth Standard. An example of a wireless LAN technology is the IEEE 802.11x Wireless LAN Standards.

Typical short-range network devices include, but are not limited to, mobile telephones, personal or laptop computers, and personal electronic devices such as personal digital assistants (PDA), pagers, portable-computing devices, or medical devices. Each Bluetooth device includes application and operating system programs including service discovery protocols which are designed to discover other Bluetooth devices (i.e. peer devices) as they enter or leave the communication range of the Bluetooth device. Devices can dynamically join or leave a Bluetooth network.

Personal Healthcare systems are increasingly using wireless communication. Typically, wireless medical sensors and devices are used to obtain, process or display telemetric data of the patient such as patient's weight, blood pressure, vital functions, and the like. The wireless medical sensors and devices use radio links for communication and transmission of data to other devices within the network or external care providers. For example, the monitoring systems typically include several devices (such as sensors, measurement devices, displays, servers, communication devices which connect to external medical services) which have to communicate with each other to provide the desired service.

Setting up such a wireless system is a difficult task. Manual configuration is time-consuming and requires complex interactions and detailed knowledge of communication systems and networking. The user, for example, has to configure network type, network name, network addresses and security parameters (e.g. PINs) for all involved devices. While professionals are typically available to set up and maintain commercial networks in hospitals and health care facilities, this support is not necessarily available for home networks. When it is available, the costs are high. Manual configuration and set up are often not feasible by the patients, as many patients are elderly people who are not technologically literate.

In addition, when a non-expert user controls the network set up and configuration, an unauthorized user might connect to the devices and make possible the misuse of the system.

In EP 1 024 626 A1 there is disclosed a method, apparatus and communication system for the exchange of information in networked pervasive environments. The disclosed method, apparatus and communication system allows achieving an authenticated and secure communication session. The method comprises using a first device and at least a remote second device. A unidirectional wireless communication channel between the first device and the remote second device is initiated and a sequence of data is communicated from the first device to the second device via this unidirectional wireless communication channel to furnish the remote second device with encryption information. In response to this, an encrypted message is sent via a wireless broadcast medium from the second device to the first device by using said encryption information for encryption.

In EP 1 220 501 A2 there is presented a group creation method for wireless communication terminals. This method comprises detecting a physical contact between users of wireless terminals and identifying a group therefrom. The step of detecting the physical contact comprises the step of transferring a signal via the physical contact between the users of the wireless terminals using a first communication circuit in the wireless terminals. The step of transferring a signal comprises the steps of generating the signal in the circuit, transferring the generated signals to a body of the user and to a body of the second user wherein both users are physically connected. The step of establishing the group comprises the step of confirming the establishment of the group between the users of the wireless terminals using a message that is transmitted using the second circuit.

Other solutions use semi-automatic configuration and setup of the wireless communication system. The semi-automatic solutions require additional dedicated setup devices, similar to the manual setup solution, and are complex to use. The present application provides new and improved apparatuses and methods which overcome the above-referenced problems and others.

In accordance with one aspect, a system for automatic configuration and set up of an ad hoc wireless medical network as set forth in claim 1 is disclosed. Wireless peer devices each includes a peer body coupled (BCC) interface module for authenticating a patient and transmitting the device identification of a selected peer device via the patient body, and a short-range network interface module for setting up a communication connection between the peer devices. An active identification device, which is linked to the patient, configured to authenticate each selected peer device and automatically associates each selected peer device with the patient. A patient body coupled communication (BCC) interface module is coupled with the patient for transmitting network parameters from the active identification device to the peer devices, configured to communicate patient identification from the active identification device via a patient body to the peer body coupled communication interface module of each selected peer device and to receive the device identification from each selected peer body coupled communication interface module via the patient body when the patient body coupled communication interface is temporarily coupled to the selected peer body coupled communication interface module.

In accordance with another aspect, an adapter for wirelessly interconnecting peer devices, which each includes a short-range wireless interface unit, as set forth in claim 7 is disclosed. A first portion is coupled to a person and includes a person BCC interface for transmitting person identification and network configuration parameters via a person body. A second portion is associated with each individual peer device and respective short-range wireless interface unit, and includes a peer BCC interface for transmitting device identification via the person body when the person and a selected peer device are temporarily coupled and a communication controller which is configured to transmit the device identification of the selected peer device to the first portion via a respective peer body coupled communication interface.

In accordance with another aspect, a method as set forth in claim 9 is disclosed. A BCC interface, connected to a patient, is temporarily linked to BCC interface modules connected to first and second peer devices. The first and second peer devices are automatically connected into a wireless network in response to the first and second peer devices being linked to the patient BCC interface, wherein the step of automatically connecting includes receiving patient identification via a patient body at the first device; receiving device identification of the first peer device via the patient body at the patient body coupled communication interface; and associating the first peer device identification with the patient identification.

In accordance with other aspects a wireless peer device as claimed in claim 6 and an active identification device as claimed in claim 12 are disclosed.

Still further advantages and benefits of the present application will become apparent to those of ordinary skill in the art upon reading and understanding the following detailed description of the preferred embodiments.

The application may take form in various components and arrangements of components, and in various steps and arrangements of steps. The drawings are only for purposes of illustrating the preferred embodiments and are not to be construed as limiting the application.
FIGURE 1 is a diagrammatic illustration of an identification system;
FIGURE 2 shows a process flow of the identification system;
FIGURE 3 shows a process flow of the second identification system.

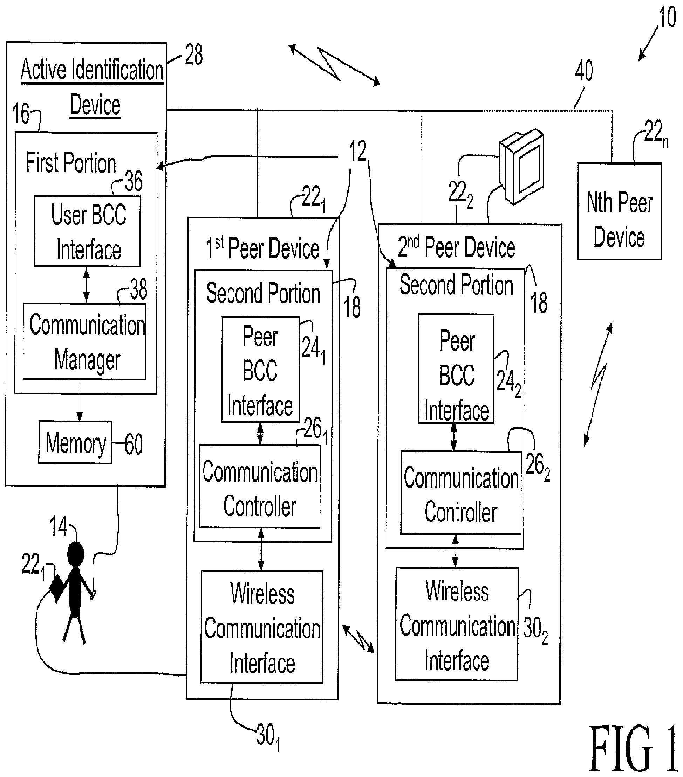

With reference to FIGURE 1, a medical system **10** includes a network adapter **12** which associates various devices with a user or patient **14** and configures the associated devices into a network. The network adapter **12** includes a first portion **16** which is linked to the patient **14** and a second portion **18** which is included with each of first, second, ..., n^{th} peer devices **22₁, 22₂, ..., 22ₙ,** such as wireless medical devices, communication devices, and the like.

The examples of the peer devices include a sensor node **22₁** disposed on the patient **14** to monitor vital signs such as electrocardiographic (ECG) data, heart rate, respiratory rate, respiratory cycle, blood pressure, or so forth; and a communication device, such as an illustrated home television set **22₂,** a remote control, a VCR, a cable box, a computer, and the like. The illustrated devices are examples, and those skilled in the art can readily include additional or other devices such as high resolution sensors, bedside monitors, ventilators, and the like that can be coupled into the network. Moreover, the devices can be arranged into the network on an ad hoc basis by adding or removing medical or communication devices.

Each second portion **18** includes a first or peer body coupled communication (BCC) interface device or module or unit **24₁, 24₂, ... , 24ₙ** capable of wireless communication via near-feld body-communication technology, which is based on capacitive coupling to the patient **14**, a communication controller **26₁, 26₂, ... , 26ₙ** for establishing wireless communications with a patient's active identification device or means or module **28**, as described below, and controlling a wireless short-range communication interface device or module or unit **30₁, 30₂, ..., 30ₙ** such as a Bluetooth communication interface for establishing wireless communications between the peer devices. A description of the Bluetooth communication protocol and device operation principles is found, for example, in Bluetooth Special Interest Group, Specification of the Bluetooth Standard, version 1.0B, volumes 1 and 2, December 1999 and Bluetooth Specification, Version 2.0, November 2004. Of course, it is contemplated that the peer device can use other short-range technologies such as IEEE 802.15.4 ZigBee, 802.11 WLAN and the like short-range communication technologies. Each Bluetooth interface device **30₁, 30₂, ... , 30ₙ** includes application and operating system programs designed to find other Bluetooth devices as the other devices enter and leave the communication range of the network.

The active identification device or means or module **28** is attached to or associated with the patient or user or patient's body **14** as a band at a wrist, leg, built into a watch, an ID card, or the like. Alternatively, the active identification device **28** is a noncontact device and is disposed in a close proximity, e.g. within 10cm, of the patient's body **14**. The active identification device **28** utilizes a near-field body-communication technology to communicate with the peer devices **22₁, 22₂, ... , 22ₙ** when the patient **14** is coupled to a selected peer device. The active identification device **28** includes a body coupled second or patient or user communication (BCC) interface **36** and a communication manager **38**. As described in detail below, when the user **14** touches, for example, at least one of the first and second devices **22₁, 22₂,** the communication controllers **26₁, 26₂** of each touched device **22₁**, **22₂** and the active identification device communication manager **38** communicate with each other via the peer BCC interface devices **24₁, 24₂** and patient BCC interface device **36.** After verifying the user's authorization, the first and second devices **22₁, 22₂** send the corresponding device data to the active identification device **28**, after which the network configuration parameters are received by the touched first and second peer devices **22₁, 22₂** from the active identification device **28**. The communication controllers **26₁, 26₂** and wireless communication interface modules **30₁, 30₂** of each first and second peer device **22₁, 22₂** are activated and initiate establishing a wireless ad-hoc network **40**. After the network **40** is established, the application data is exchanged among the first and second peer devices **22₁, 22₂,** e.g. the network members. A third or additional wireless device can join an existing established wireless network **40** when the user **14** touches the **n^{th}** device (not shown) and any device belonging to the established network **40** such as the first and second peer devices **22₁, 22₂** of the example above.

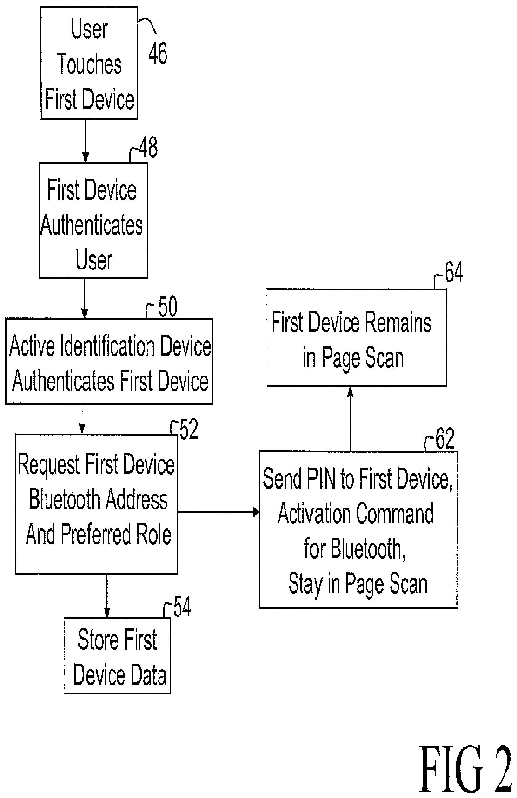

With continuing reference to FIGURE 1 and further reference to FIGURE 2, the user **14** touches **46** the first device **22₁**. The active identification device communication manager **38** communicates with the first device communication controller **26₁** via body coupled communications. The first device **22₁** authenticates **48** the active identification device **28** and the user, e.g. the first device **22₁** reads the patient's identification to avoid usage by unauthorized users. The active identification device **28** authenticates **50** the first device **22₁** via body coupled communications. After successful mutual authentication, the active identification device communication manager **38** requests **52** the first device **22₁** to transmit a first device Bluetooth Address and Preferred Bluetooth Role such as master, slave or both. The first device data is stored **54** in an active identification device memory **60** for a fixed period of Bluetooth data timeout time **Tₒᵤₜ**. The active identification device communication controller **38** sends **62** to the first device communication controller **26₁** a pin number (PIN) to be used during the network connection establishment, a command message to activate the first peer device Bluetooth interface **28₁**, and a command for the first device **22₁** to remain **64** in a page scan mode for a page scan duration time **T**_{pg_scan}.

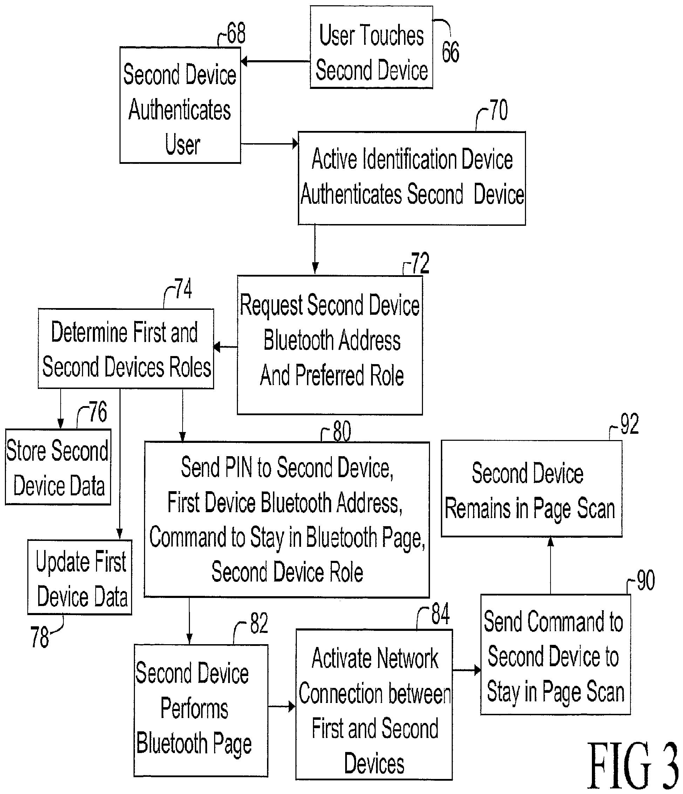

With continuing reference to FIGURE 1 and further reference to FIGURE 3, to connect the second device **22₂** into the network **40**, the user **14** touches **66** the second device **22₂**. The active identification device communication manager **38** communicates with the second device communication controller **26₂** via body coupled communications. The second device **22₂** authenticates **68** the active identification device **28** and the user to avoid usage by unauthorized users. The active identification device **28** authenticates **70** the second device **22₂.** After successful mutual authentication, the active identification device communication manager **38** requests **72** the second device communication controller **26₂** to transmit a second device Bluetooth Address and Preferred Bluetooth Role such as master, slave or both. The active identification device communication controller **38** determines **74** which of the first and second devices **22₁, 22₂** is the master of the network **40**, e.g. the network roles are assigned to the first and second devices. The data of the second device **22₂**, including at least its role as a master or a slave, is stored **76** in the active identification device memory **60** for a fixed period of Bluetooth data timeout time **Tₒᵤₜ**. The device role, e.g. master or slave, of the first device **22₁** is updated **78** in the active identification device memory **60** if required. It is important to keep the data of the master of the connection - at least this data needs to be stored in the active identification device memory **60**.

The active identification device communication manager **38** sends **80** to the second device communication controller **26₂** a command message to perform a Bluetooth page to the first device **22₁**. More specifically, the active identification device **28** sends **80** the second device communication controller **26₂** a message including the first device Bluetooth address, pin number PIN, a Bluetooth page duration time **T_{pg}** and the second device network role such as a master or a slave. The second device wireless interface **30₂** performs **82** the Bluetooth page while the first device wireless interface **30₁** is in the page scan mode, i.e. the Bluetooth interface connection set up between the first and second devices **22₁**, **22₂** is activated **84**. This results in a network connection establishment between the first and second devices for a first-second devices connection establishment duration time **T_{D1-D2}**.

The active identification device communication controller **38** sends **90** to the second device **22₂** a request to stay in the page scan mode after the connection between the first and second devices **22₁**, **22₂** has been established. The second device **22₂** remains **92** in the page scan mode for a time equal to the difference between the Bluetooth data timeout **Tₒᵤₜ** and the first-second devices connection establishment duration time **T_{D1-D2}**.

Of course, it is also contemplated that the user **14** can touch the first and second peer devices **22₁, 22₂** at the same time to initiate interconnection of the first and second devices **22₁, 22₂** into the network **40**.

If the Bluetooth data timeout time **Tₒᵤₜ** of the active identification device **28** times out, the data of the first and second devices **22₁, 22₂** is deleted. The connection of the two devices remains unless terminated by other actions or control software on the devices.

To connect the third device (not shown) into the established network **40** between the first and second devices **22₁, 22₂**, the user **14** touches the third device. The following situations are distinguished:
(a) The second device **22₂** is in the page scan mode: The user touches the third device. The connection is established similar to the establishment of the second device connection described above.
(b) The second device **22₂** is in the page scan mode: The connection can be established by the user touching the network member, and subsequently the third device.
(c) The second device **22₂** page scan duration time **T_{pg_scan}** is expired: The connection can be established by the user touching the third device, and subsequently the network member **22₁** or **22₂**.
(d) The second device page scan duration time **T_{pg_scan}** is expired: The connection can be established by the user touching the network member **22₁** or **22₂**, and subsequently the third device.

The system takes into account whether the touched network member is the master or one of the slaves and whether the network supports scatternets or not.

The Bluetooth page time **T_{pg}** and page scan time **T_{pg_scan}** are the timeouts for the devices performing Bluetooth Page or Bluetooth Page Scan, respectively. During the procedure to connect two devices that are not connected to any network, the Bluetooth page time **T_{pg}** and page scan time **T_{pg-scan}** can be different due to some delays on the body coupled communication. The active identification device **28** is aware of the introduced delay, e.g. using an internal clock, and modifies accordingly the Bluetooth page time T**_{pg}** and page scan time **T_{pg_scan}** in the command message sent to the devices. If the active identification device **28** determines that there is no delay, the active identification device **28** sets the Bluetooth page time **T_{pg}** to be equal to the page scan time **T_{pg_scan}**. The active identification device **28** sets the values of the Bluetooth page time **T_{pg}** and page scan time **T_{pg_scan}** according to the application. For example, a value of the Bluetooth data timeout **Tₒᵤₜ** is selected to be from about **10** seconds to about 60 seconds. In one embodiment, the value of the Bluetooth data timeout **Tₒᵤₜ** is greater than or equal to the Bluetooth page time **T_{pg}**. In one embodiment, the equal value such as 10 seconds for the Bluetooth data timeout **Tₒᵤₜ**, the Bluetooth page time **T_{pg}** and page scan time T**_{pg_scan}** is selected. E.g., it is assumed it takes about 10 seconds to touch two devices sequentially.

In this manner, intuitive automatic and secure configuration and set-up of wireless networks is accomplished where devices are connected when the user touches the devices simultaneously or subsequently. By mutual authentication between each of the devices **22₁, 22₂,** ... , **22ₙ** and the active identification device **28** worn by the user **14**, who is touching the devices to be connected, the connection set-up is limited to authorized persons only. The identified user can connect only to the devices particularly designated for use by the identified user when he/she touches the devices.

As another example, using Bluetooth discovery directly, the user might discover the available devices. The user then selects from a list of all found devices. In this case all devices being in Bluetooth range would be discovered. (This is not really a user friendly solution.)

In the manner described above, the active identification unit is used as a middleman for storage and transmission of necessary information to set up a network connection. As the BCC interface needs less power as compared to the Bluetooth interface, using BCC interface for connection set up saves power compared to using mechanisms of Bluetooth directly as the Bluetooth interface can sleep until needed.

The method and apparatus described above can be applied in all domains where wireless ad hoc networks are being deployed and to all types of wireless technologies such as Bluetooth, WLAN (IEEE 802.11), ZigBee (IEEE 802.15.4) and the like. The application areas include: (a) Easy and secure set-up of wireless networks for Personal Area Networks as in PHC, including user-specific configuration of devices; (b) Association of body-worn devices with peripheral devices, for example ECG sensor and bedside monitor for home monitoring of vital signs, integration of wireless scale to body area network for personal healthcare applications; and (e) Application in consumer-related application, allowing easy integration of home monitoring functionality into in-home networks.

It is also contemplated that some devices, particularly communication devices, can be on more than one local network. In this manner, two patients in the same household can communicate their medical information to a central location with the same communication device.

The above has been described with reference to the preferred embodiments. Modifications and alterations may occur to others upon a reading and understanding of the preceding detailed description.

## Claims

1. A system (10) for automatic configuration and set up of an ad hoc wireless medical network (40) comprising:
wireless peer devices (22₁, 22₂, ... , 22ₙ) which each includes:
a peer body coupled communication interface module (24₁, 24₂,..., 24ₙ) for authenticating a patient and transmitting device identification of a selected peer device (22₁, 22₂, ... , 22ₙ) via the patient body, and
a short-range network interface module (30₁, 30₂, ..., 30ₙ) for setting up a communication connection between the peer devices (22₁, 22₂, ..., 22ₙ); and
an active identification device (28), linked to the patient (14), which active identification device (28) is configured to authenticate each selected peer device (22₁, 22₂, ..., 22ₙ) and automatically associate each selected peer device (22₁, 22₂,..., 22ₙ) with the patient (14), the active identification device including:
a patient body coupled communication interface module (36), coupled with the patient (14) for transmitting network parameters from the active identification device (28) to the peer devices (22₁, 22₂, ..., 22ₙ), configured to communicate patient identification from the active identification device (28) via a patient body to the peer body coupled communication interface module (24₁, 24₂,..., 24ₙ) of each selected peer device (22₁, 22₂, ..., 22ₙ) and to receive the device identification from each selected peer body coupled communication interface module (24₁, 24₂,..., 24ₙ) via the patient body when the patient body coupled communication interface (36) is temporarily coupled to the selected peer body coupled communication interface module (24₁, 24₂,..., 24ₙ).

2. The system as set forth in claim 1, wherein the active identification device (28) further includes:
a communication manager (38) which is configured to automatically associate the transmitted device identification with the patient identification and assign network configuration parameters to each selected peer device to configure the selected peer devices (22₁, 22₂, ... , 22ₙ) into the network (40).

3. The system as set forth in claim 2, wherein each peer device (22₁, 22₂, ..., 22ₙ) further includes:
a communication controller (26₁, 26₂, ..., 26ₙ) which is configured to transmit the device identification to the communication manager (38) and receive the network configuration parameters from the communication manager (38), the communication controller (26₁, 26₂, ... , 26ₙ) and communication manager (38) configured to communicate with one another via the selected peer body coupled communication interface module and patient body coupled communication interface module.

4. The system as set forth in claim 3, wherein the communication manager (38) is configured to configure the short-range network interface modules (30₁, 30₂, ..., 30ₙ) of the selected peer devices (22₁, 22₂, ..., 22ₙ) to establish network connection between the selected peer devices (22₁, 22₂, ... , 22ₙ).

5. The system as set forth in claim 2, wherein the active identification device (28) further includes:
a memory (60) which is configured to store the transmitted device identification and assigned network parameters of the selected peer devices for a data timeout time (Tₒᵤₜ).

6. A wireless peer device (22₁, 22₂, ..., 22ₙ) for use in the system of claim 1, the wireless peer device including:
said peer body coupled communication interface module (24₁, 24₂,..., 24ₙ) and said short range network interface module (30₁, 30₂, ..., 30ₙ); and
a communication controller (26₁, 26₂, ..., 26ₙ) which is configured to transmit the device identification to a communication manager (38) comprised in the active identification device and receive the network configuration parameters from the communication manager (38), the communication controller (26₁, 26₂, ... , 26ₙ) and communication manager (38) are configured to bidirectionally communicate with one another via a patient body.

7. An adapter (12) for wirelessly interconnecting peer devices (22₁, 22₂, ..., 22ₙ), which each includes a short-range wireless interface unit (30₁, 30₂, ..., 30ₙ), the adapter (12) comprising:
a first portion (16) coupled to a person (14) and including a person body coupled communication interface (36) for transmitting person identification and network configuration parameters via a person body; and
a second portion (18) associated with each individual peer device (22₁, 22₂, ..., 22ₙ) and respective short-range wireless interface unit (30₁, 30₂, ..., 30ₙ), which second portion (18) includes a peer body coupled communication interface (24₁, 24₂,..., 24ₙ) for transmitting device identification via the person body when the person (14) and a selected peer device (22₁, 22₂, ..., , 22ₙ) are temporarily coupled and a communication controller (26₁, 26₂, ..., 26ₙ) which is configured to transmit the device identification of the selected peer device (22₁, 22₂, ..., 22ₙ) to the first portion (16) via a respective peer body coupled communication interface (24₁, 24₂, ..., 24ₙ).

8. The adapter as set forth in claim 7, wherein the first portion (16) further includes:
a communication manager (38) which is configured to automatically associate the transmitted device identification with the person identification and transmit the network configuration parameters to a communication controller (26₁, 26₂, ... , 26ₙ) of the selected peer device (22₁, 22₂, ... , 22ₙ) to interconnect the short-range wireless interface unit (30₁, 30₂, ..., 30ₙ) of each selected peer device (22₁, 22₂, ..., 22ₙ) so that the selected peer devices (22₁, 22₂, ..., , 22ₙ) form the network (40).

9. A method comprising:
temporarily linking a body coupled communication interface, connected to a patient, to body coupled communication interface modules connected to first and second peer devices; and
automatically connecting the first and second peer devices into a wireless network in response to the first and second peer devices being linked to the patient body coupled communication interface, wherein the step of automatically connecting includes receiving patient identification via a patient body at the first peer device;
receiving device identification of the first peer device via the patient body at the patient body coupled communication interface; and
associating the first peer device identification with the patient identification.

10. The method as set forth in claim 9, further including:
receiving patient identification via the patient body at the second peer device;
receiving device identification of the second peer device via the patient body at the patient body coupled communication interface;
associating the second peer device with the patient identification;
transmitting network configuration parameters to the second peer device; and
activating a second short-range network interface module on the second peer device.

11. The method as set forth in claim 10, wherein the step of linking includes one of:
contemporaneously linking the patient body coupled communication interface to the body coupled communication interface modules connected to the first and second peer devices, and
linking the patient body coupled communication interface to the body coupled communication interface module connected to the second peer device subsequent to linking the patient body coupled communication interface to the body coupled communication interface module connected to the first peer device.

12. An active identification device (28) for automatically connecting first and second peer devices into a wireless network, said active identification device (28) being configured to
temporarily link a body coupled communication interface, connected to a patient, to body coupled communication interface modules connected to the first and second peer devices; and
automatically connect the first and second peer devices into a wireless network in response to the first and second peer devices being linked to the patient body coupled communication interface;
communicate patient identification via a patient body to the first peer device;
receive device identification of the first peer device via the patient body at the patient body coupled communication interface; and
associate the first peer device identification with the patient identification.

## Patentansprüche

1. System (10) zur automatischen Konfiguration und Einrichtung eines drahtlosen, medizinischen Ad-hoc-Netzwerks (40), umfassend:
drahtlose Peer-Geräte (22₁, 22₂, ..., 22ₙ), die jeweils enthalten:
ein Peer-körpergekoppeltes Kommunikationsschnittstellenmodul (24₁, 24₂, ..., 24ₙ), um einen Patienten zu authentifizieren und eine Geräteidentifikation eines ausgewählten Peer-Geräts (22₁, 22₂, ..., 22ₙ) via den Patientenkörper zu übermitteln, und
ein Nahbereichs-Netzwerkschnittstellenmodul (30₁, 30₂, ..., 30ₙ) zur Herstellung einer Kommunikationsverbindung zwischen den Peer-Geräten (22₁, 22₂, ... , 22ₙ); sowie
eine mit dem Patienten (14) verbundene, aktive Identifikationsvorrichtung (28), wobei die aktive Identifikationsvorrichtung (28) so konfiguriert ist, dass sie jedes ausgewählte Peer-Gerät (22₁, 22₂, ..., 22n) authentifiziert und jedes ausgewählte Peer-Gerät (22₁, 22₂, ..., 22ₙ) dem Patienten (14) automatisch zuordnet, wobei die aktive Identifikationsvorrichtung enthält:
ein Patienten-körpergekoppeltes Kommunikationsschnittstellenmodul (36), das mit dem Patienten (14) gekoppelt ist, um Netzwerkparameter von der aktiven Identifikationsvorrichtung (28) zu den Peer-Geräten (22₁, 22₂, ..., 22ₙ) zu übertragen, und so konfiguriert ist, dass es eine Patientenidentifikation von der aktiven Identifikationsvorrichtung (28) via einen Patientenkörper zu dem Peer-körpergekoppelten Kommunikationsschnittstellenmodul (24₁, 24₂,..., 24ₙ) jedes ausgewählten Peer-Geräts (22₁, 22₂, ..., 22ₙ) überträgt und die Geräteidentifikation von jedem ausgewählten Peer-körpergekoppelten Kommunikationsschnittstellenmodul (24₁, 24₂, ..., 24ₙ) via den Patientenkörper empfängt, wenn die Patienten-körpergekoppelte Kommunikationsschnittstelle (36) temporär mit dem ausgewählten Peer-körpergekoppelten Kommunikationsschnittstellenmodul (24₁, 24₂, ..., 24ₙ) gekoppelt ist.

2. System nach Anspruch 1, wobei die aktive Identifikationsvorrichtung (28) weiterhin enthält:
einen Communication Manager (38), der so konfiguriert ist, dass er die übermittelte Geräteidentifikation der Patientenidentifikation automatisch zuordnet und jedem ausgewählten Peer-Gerät Netzwerkkonfigurationsparameter zuweist, um die ausgewählten Peer-Geräte (22₁, 22₂, ..., 22ₙ) in das Netzwerk (40) zu konfigurieren.

3. System nach Anspruch 2, wobei jedes Peer-Gerät (22₁, 22₂, ..., 22ₙ) weiterhin enthält:
einen Communication Controller (26₁, 26₂, ..., 26ₙ), der so konfiguriert ist, dass er die Geräteidentifikation zu dem Communication Manager (38) überträgt und die Netzwerkkonfigurationsparameter von dem Communication Manager (38) empfängt, wobei der Communication Controller (26₁, 26₂, ..., 26ₙ) und der Communication Manager (38) so konfiguriert sind, dass sie miteinander über das ausgewählte Peer-körpergekoppelte Kommunikationsschnittstellenmodul und Patienten-körpergekoppelte Kommunikationsschnittstellenmodul kommunizieren.

4. System nach Anspruch 3, wobei der Communication Manager (38) so konfiguriert ist, dass er die Nahbereichs-Netzwerkschnittstellenmodule (30₁, 30₂, ..., 30ₙ) der ausgewählten Peer-Geräte (22₁, 22₂, ..., 22ₙ) konfiguriert, um eine Netzwerkverbindung zwischen den ausgewählten Peer-Geräten (22₁, 22₂, ..., 22ₙ) herzustellen.

5. System nach Anspruch 2, wobei die aktive Identifikationsvorrichtung (28) weiterhin enthält:
einen Speicher (60), der so konfiguriert ist, dass er die übertragene Geräteidentifikation sowie zugewiesene Netzwerkparameter der ausgewählten Peer-Geräte für eine Daten-Timeout-Zeit (Tₒᵤₜ) speichert.

6. Drahtloses Peer-Gerät (22₁, 22₂, ..., 22ₙ) zur Verwendung in dem System nach Anspruch 1, wobei das drahtlose Peer-Gerät enthält:
das Peer-körpergekoppelte Kommunikationsschnittstellenmodul (24₁, 24₂, ..., 24ₙ) und das Nahbereichs-Netzwerkschnittstellenmodul (30₁, 30₂, ..., 30ₙ); sowie
einen Communication Controller (26₁, 26₂, ..., 26ₙ), der so konfiguriert ist, dass er die Geräteidentifikation zu einem in der aktiven Identifikationsvorrichtung enthaltenen Communication Manager (38) überträgt und die Netzwerkkonfigurationsparameter von dem Communication Manager (38) empfängt, wobei der Communication Controller (26₁, 26₂, ..., 26ₙ) und der Communication Manager (38) so konfiguriert sind, dass sie miteinander via einen Patientenkörper bidirektional kommunizieren.

7. Adapter (12) zur drahtlosen Verbindung von Peer-Geräten (22₁, 22₂, ..., 22ₙ), die jeweils eine drahtlose Nahbereichs-Schnittstelleneinheit (30₁, 30₂, ..., 30ₙ) enthalten, wobei der Adapter (12) umfasst:
einen ersten Abschnitt (16), der mit einer Person (14) gekoppelt ist und eine Personen-körpergekoppelte Kommunikationsschnittstelle (36) enthält, um Personenidentifikations- und Netzwerkkonfigurationsparameter via einen Personenkörper zu übertragen; sowie
einen zweiten Abschnitt (18), der jedem einzelnen Peer-Gerät (22₁, 22₂, ..., 22ₙ) und der jeweiligen drahtlosen Nahbereichs-Schnittstelleneinheit (30₁, 30₂, ..., 30ₙ) zugeordnet ist, wobei der zweite Abschnitt (18) eine Peer-körpergekoppelte Kommunikationsschnittstelle (24₁, 24₂, ..., 24ₙ), um die Geräteidentifikation via den Personenkörper zu übertragen, wenn die Person (14) und ein ausgewähltes Peer-Gerät (22₁, 22₂, ..., 22ₙ) temporär gekoppelt sind, sowie einen Communication Controller (26₁, 26₂, ..., 26ₙ) enthält, der so konfiguriert ist, dass er die Geräteidentifikation des ausgewählten Peer-Geräts (22₁, 22₂, ..., 22ₙ) via eine jeweilige Peer-körpergekoppelte Kommunikationsschnittstelle (24₁, 24₂, ..., 24ₙ) zu dem ersten Abschnitt (16) überträgt.

8. Adapter nach Anspruch 7, wobei der erste Abschnitt (16) weiterhin enthält:
einen Communication Manager (38), der so konfiguriert ist, dass er die übertragene Geräteidentifikation der Personenidentifikation automatisch zuordnet und die Netzwerkkonfigurationsparameter zu einem Communication Controller (26₁, 26₂, ..., 26ₙ) des ausgewählten Peer-Geräts (22₁, 22₂, ..., 22ₙ) überträgt, um die drahtlose Nahbereichs-Schnittstelleneinheit (30₁, 30₂, ..., 30ₙ) jedes ausgewählten Peer-Geräts (22₁, 22₂, ..., 22ₙ) untereinander zu verbinden, so dass die ausgewählten Peer-Geräte (22₁, 22₂, ..., 22ₙ) das Netzwerk (40) bilden.

9. Verfahren, wonach:
eine mit einem Patienten verbundene, körpergekoppelte Kommunikationsschnittstelle temporär mit körpergekoppelten Kommunikationsschnittstellenmodulen, die mit einem ersten und zweiten Peer-Gerät verbunden sind, verlinkt werden; und
das erste und zweite Peer-Gerät in Reaktion darauf, dass das erste und zweite Peer-Gerät mit der Patienten-körpergekoppelten Kommunikationsschnittstelle verlinkt sind, automatisch an ein drahtloses Netzwerk angeschlossen werden, wobei der Schritt des automatischen Anschließens beinhaltet:
das Empfangen einer Patientenidentifikation via einen Patientenkörper an dem ersten Peer-Gerät;
das Empfangen einer Geräteidentifikation des ersten Peer-Geräts via den Patientenkörper an der Patienten-körpergekoppelten Kommunikationsschnittstelle; und
Zuordnen der Identifikation des ersten Peer-Geräts zu der Patientenidentifikation.

10. Verfahren nach Anspruch 9, wonach weiterhin:
die Patientenidentifikation via den Patientenkörper an dem zweiten Peer-Gerät empfangen wird;
die Geräteidentifikation des zweiten Peer-Geräts via den Patientenkörper an der Patienten-körpergekoppelten Kommunikationsschnittstelle empfangen wird;
das zweite Peer-Gerät der Patientenidentifikation zugeordnet wird;
Netzwerkkonfigurationsparameter zu dem zweiten Peer-Gerät übertragen werden; und
ein zweites Nahbereichs-Netzwerkschnittstellenmodul auf dem zweiten Peer-Gerät aktiviert wird.

11. Verfahren nach Anspruch 10, wobei der Schritt des Verlinkens beinhaltet:
kontemporäres Verlinken der Patienten-körpergekoppelten Kommunikationsschnittstelle mit den, mit dem ersten und zweiten Peer-Gerät verbundenen, körpergekoppelten Kommunikationsschnittstellenmodulen; oder
Verlinken der Patienten-körpergekoppelten Kommunikationsschnittstelle mit dem, mit dem zweiten Peer-Gerät verbundenen, körpergekoppelten Kommunikationsschnittstellenmodul im Anschluss an das Verlinken der Patienten-körpergekoppelten Kommunikationsschnittstelle mit dem, mit dem ersten Peer-Gerät verbundenen, körpergekoppelten Kommunikationsschnittstellenmodul.

12. Aktive Identifikationsvorrichtung (28), um das erste und zweite Peer-Gerät automatisch an ein drahtloses Netzwerk anzuschließen, wobei die aktive Identifikationsvorrichtung (28) so konfiguriert ist, dass sie
eine mit einem Patienten verbundene, körpergekoppelte Kommunikationsschnittstelle temporär mit körpergekoppelten Kommunikationsschnittstellenmodulen, die mit dem ersten und zweiten Peer-Gerät verbunden sind, verlinkt; und
das erste und zweite Peer-Gerät in Reaktion darauf, dass das erste und zweite Peer-Gerät mit der Patienten-körpergekoppelten Kommunikationsschnittstelle verlinkt sind, automatisch an ein drahtloses Netzwerk anschließt;
eine Patientenidentifikation via einen Patientenkörper zu dem ersten Peer-Gerät überträgt;
eine Geräteidentifikation des ersten Peer-Geräts via den Patientenkörper an der Patienten-körpergekoppelten Kommunikationsschnittstelle empfängt; und
die Identifikation des ersten Peer-Geräts der Patientenidentifikation zuordnet.

## Revendications

1. Système (10) pour la configuration automatique et le paramétrage d'un réseau médical sans fil ad hoc (40) comprenant :
des dispositifs homologues sans fil (22₁, 22₂, ..., 22ₙ) qui comportent chacun :
un module d'interface de communication couplé à un corps homologue (24₁, 24₂, ..., 24ₙ) pour l'authentification d'un patient et la transmission de l'identification de dispositif d'un dispositif homologue sélectionné (22₁, 22₂, ..., 22ₙ) via le corps du patient, et
un module d'interface réseau à courte portée (30₁, 30₂, ..., 30ₙ) pour paramétrer une connexion de communication entre les dispositifs homologues (22₁, 22₂, ..., 22ₙ) ; et
un dispositif actif d'identification (28), relié au patient (14), lequel dispositif actif d'identification (28) est configuré pour authentifier chaque dispositif homologue sélectionné (22₁, 22₂, ..., 22ₙ) et associer automatiquement chaque dispositif homologue sélectionné (22₁, 22₂, ..., 22ₙ) au patient (14), le dispositif actif d'identification comportant :
un module d'interface de communication couplé au corps d'un patient (36), couplé avec le patient (14) pour transmettre des paramètres de réseau du dispositif actif d'identification (28) aux dispositifs homologues (22₁, 22₂, ..., 22n), configuré pour communiquer l'identification de patient du dispositif actif d'identification (28) via un corps de patient au module d'interface de communication couplé au corps homologue (24₁, 24₂, ..., 24ₙ) de chaque dispositif homologue sélectionné (22₁, 22₂, ..., 22ₙ) et pour recevoir l'identification de dispositif de chaque module d'interface de communication couplé au corps homologue sélectionné (24₁, 24₂, ..., 24ₙ) via le corps de patient lorsque l'interface de communication couplée au corps de patient (36) est temporairement couplée au module d'interface de communication couplé au corps homologue sélectionné (24₁, 24₂, ..., 24ₙ).

2. Système selon la revendication 1, dans lequel le dispositif actif d'identification (28) comporte en outre :
un gestionnaire de communication (38) qui est configuré pour associer automatiquement l'identification de dispositif transmise à l'identification de patient et attribuer des paramètres de configuration de réseau à chaque dispositif homologue sélectionné pour configurer les dispositifs homologues sélectionnés (22₁, 22₂, ..., 22ₙ) dans le réseau (40).

3. Système selon la revendication 2, dans lequel chaque dispositif homologue (22₁, 22₂, ..., 22ₙ) comporte en outre :
un contrôleur de communication (26₁, 26₂, ..., 26ₙ) qui est configuré pour transmettre l'identification de dispositif au gestionnaire de communication (38) et recevoir les paramètres de configuration de réseau du gestionnaire de communication (38), le contrôleur de communication (26₁, 26₂, ..., 26ₙ) et le gestionnaire de communication (38) étant configurés pour communiquer entre eux via le module d'interface de communication couplé au corps homologue sélectionné et le module d'interface de communication couplé au corps de patient.

4. Système selon la revendication 3, dans lequel le gestionnaire de communication (38) est configuré pour configurer les modules d'interface réseau à courte portée (30₁, 30₂, ..., 30ₙ) des dispositifs homologues sélectionnés (22₁, 22₂, ..., 22ₙ) afin d'établir une connexion de réseau entre les dispositifs homologues sélectionnés (22₁, 22₂, ..., 22ₙ).

5. Système selon la revendication 2, dans lequel le dispositif actif d'identification (28) comporte en outre :
une mémoire (60) qui est configurée pour stocker l'identification de dispositif transmise et les paramètres de réseau attribués des dispositifs homologues sélectionnés pendant un délai d'attente (Tₒᵤₜ) de données.

6. Dispositif homologue sans fil (22₁, 22₂, ..., 22ₙ) destiné à être utilisé dans le système selon la revendication 1, le dispositif homologue sans fil comportant :
ledit module d'interface de communication couplé au corps homologue (24₁, 24₂, ..., 24ₙ) et ledit module d'interface réseau à courte portée (30₁, 30₂, ..., 30ₙ) ; et
un contrôleur de communication (26₁, 26₂, ..., 26ₙ) qui est configuré pour transmettre l'identification de dispositif à un gestionnaire de communication (38) inclus dans le dispositif actif d'identification et recevoir les paramètres de configuration de réseau du gestionnaire de communication (38), le contrôleur de communication (26₁, 26₂, ..., 26n) et le gestionnaire de communication (38) sont configurés pour communiquer de manière bidirectionnelle l'un avec l'autre via un corps de patient.

7. Adaptateur (12) pour interconnecter sans fil des dispositifs homologues (22₁, 22₂, ..., 22ₙ), qui comportent chacun une unité d'interface sans fil à courte portée (30₁, 30₂, ..., 30ₙ), l'adaptateur (12) comprenant :
une première partie (16) couplée à une personne (14) et comportant une interface de communication couplée à un corps de personne (36) pour transmettre une identification de personne et des paramètres de configuration de réseau via un corps de personne ; et
une seconde partie (18) associée à chaque dispositif homologue individuel (22₁, 22₂, ..., 22ₙ) et une unité d'interface sans fil à courte portée (30₁, 30₂, ..., 30ₙ) respective, laquelle seconde partie (18) comporte une interface de communication couplée au corps homologue (24₁, 24₂, ..., 24ₙ) pour transmettre l'identification de dispositif via le corps de personne lorsque la personne (14) et un dispositif homologue sélectionné (22₁, 22₂, ..., 22ₙ) sont temporairement couplés et un contrôleur de communication (26₁, 26₂, ..., 26ₙ) qui est configuré pour transmettre l'identification de dispositif du dispositif homologue sélectionné (22₁, 22₂, ..., 22ₙ) à la première partie (16) via une interface de communication couplée au corps homologue respective (24₁, 24₂, ..., 24n).

8. Adaptateur selon la revendication 7, dans lequel la première partie (16) comporte en outre :
un gestionnaire de communication (38) qui est configuré pour associer automatiquement l'identification de dispositif transmise à l'identification de personne et transmettre les paramètres de configuration de réseau à un contrôleur de communication (26₁, 26₂, ..., 26ₙ) du dispositif homologue sélectionné (22₁, 22₂, ..., 22ₙ) pour interconnecter l'unité d'interface sans fil à courte portée (30₁, 30₂, ..., 30ₙ) de chaque dispositif homologue sélectionné (22₁, 22₂, ..., 22ₙ) de telle sorte que les dispositifs homologues sélectionnés (22₁, 22₂, ..., 22ₙ) forment le réseau (40).

9. Procédé comprenant :
la liaison temporaire d'une interface de communication couplée au corps, reliée à un patient, aux modules d'interface de communication couplés au corps reliés aux premier et second dispositifs homologues ; et
la connexion automatique des premier et second dispositifs homologues à un réseau sans fil en réponse à la liaison des premier et second dispositifs homologues à l'interface de communication couplée au corps de patient, dans lequel l'étape de connexion automatique comporte
la réception de l'identification de patient via un corps de patient au niveau du premier dispositif homologue ;
la réception de l'identification de dispositif du premier dispositif homologue via le corps de patient au niveau de l'interface de communication couplée au corps de patient ; et
l'association de la première identification de dispositif homologue à l'identification de patient.

10. Procédé selon la revendication 9, comportant en outre :
la réception de l'identification de patient via le corps de patient sur le second dispositif homologue ;
la réception de l'identification de dispositif du second dispositif homologue via le corps de patient sur l'interface de communication couplée au corps de patient ;
l'association du second dispositif homologue à l'identification de patient ;
la transmission des paramètres de configuration de réseau au second dispositif homologue ; et
l'activation d'un second module d'interface de réseau à courte portée sur le second dispositif homologue.

11. Procédé selon la revendication 10, dans lequel l'étape de liaison comporte l'une parmi :
une liaison simultanée de l'interface de communication couplée au corps de patient aux modules d'interface de communication couplés au corps connectés aux premier et second dispositifs homologues, et
une liaison de l'interface de communication couplée au corps de patient au module d'interface de communication couplé au corps connecté au second dispositif homologue suite à la liaison de l'interface de communication couplée au corps de patient au module d'interface de communication couplé au corps connecté au premier dispositif homologue.

12. Dispositif actif d'identification (28) destiné à se connecter automatiquement les premier et second dispositifs homologues à un réseau sans fil, ledit dispositif actif d'identification (28) étant configuré pour
relier temporairement une interface de communication couplée au corps, connectée à un patient, à des modules d'interface de communication couplés au corps connectés aux premier et second dispositifs homologues ; et
connecter automatiquement les premier et second dispositifs homologues dans un réseau sans fil en réponse à la liaison des premier et second dispositifs homologues à l'interface de communication couplée au corps de patient ;
communiquer l'identification de patient via un corps de patient au premier dispositif homologue ;
recevoir une identification de dispositif du premier dispositif homologue via le corps de patient sur l'interface de communication couplée au corps de patient ; et
associer l'identification de premier dispositif homologue à l'identification de patient.
